(19) Europäisches Patentamt / European Patent Office / Office européen des brevets

(11) **EP 3 250 178 B1**

(12) **EUROPÄISCHE PATENTSCHRIFT**

(45) Veröffentlichungstag und Bekanntmachung des Hinweises auf die Patenterteilung:
**01.01.2020 Patentblatt 2020/01**

(21) Anmeldenummer: **16700634.5**

(22) Anmeldetag: **15.01.2016**

(51) Int Cl.:
*A61K 8/55* (2006.01)    *A61Q 5/02* (2006.01)
*A61Q 5/12* (2006.01)    *A61Q 19/10* (2006.01)
*A61K 8/60* (2006.01)

(86) Internationale Anmeldenummer:
**PCT/EP2016/050744**

(87) Internationale Veröffentlichungsnummer:
**WO 2016/120094 (04.08.2016 Gazette 2016/31)**

(54) **VERWENDUNG VON EINPHASIGEN TENSID/ÖL/WASSER ZUBEREITUNGEN IN KOSMETISCHEN REINIGUNGSMITTELN**

USE OF SINGLE PHASE TENSIDE/OIL/WATER PREPARATIONS IN COSMETIC CLEANSING COMPOSITIONS

UTILISATION DE PRÉPARATIONS D'EAU/HUILE/TENSIOACTIF MONOPHASÉES DANS DES PRODUITS DE NETTOYAGE COSMÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.01.2015 EP 15152632**

(43) Veröffentlichungstag der Anmeldung:
**06.12.2017 Patentblatt 2017/49**

(73) Patentinhaber: **BASF SE**
**67056 Ludwigshafen am Rhein (DE)**

(72) Erfinder:
- **HLOUCHA, Matthias**
  **50677 Köln (DE)**
- **KÜSTERS, Esther**
  **40699 Erkrath (DE)**
- **SCHORB, Jasmin**
  **40789 Monheim (DE)**
- **SEIDLER, Stefanie**
  **40597 Düsseldorf (DE)**

(74) Vertreter: **BASF IP Association**
**BASF SE**
**G-FLP-C006**
**67056 Ludwigshafen (DE)**

(56) Entgegenhaltungen:
WO-A2-2013/149853    DE-A1-102008 024 570
US-A1- 2013 012 423    US-B1- 6 221 389

- **Anonymous: "Neutralizing Shampoo", , 1. Oktober 2010 (2010-10-01), XP055256722, Internet Gefunden im Internet: URL:http://www.gnpd.com/sinatra/recordpage /1907709/from_search/kb7SQMQYz2/with_sort/ 2/ [gefunden am 2016-03-09]**
- **Anonymous: "Shampoo", MINTEL GNPD, 1. November 2014 (2014-11-01), Seiten 1-3, XP055204351, INTERNET Gefunden im Internet: URL:http://www.gnpd.com/sinatra/recordpage /2785715/from_search/DB12KUyFkX/ [gefunden am 2015-07-23]**

Bemerkungen:
Die Akte enthält technische Angaben, die nach dem Eingang der Anmeldung eingereicht wurden und die nicht in dieser Patentschrift enthalten sind.

**Beschreibung**

**Gebiet der Erfindung**

Die Erfindung liegt auf dem Gebiet der kosmetischen Reinigungsmittel und betrifft die Verwendung von einphasigen Tensid/Öl/Wasser-Zubereitungen als konditionierend wirkendes Mittel in kosmetischen Reinigungsmitteln, ausgewählte Tensid/Öl/Wasser-Zubereitungen sowie milde kosmetische Reinigungsmittel für Haut und Haar enthaltend derartige Tensid/Öl/Wasser-Zubereitungen und ein Verfahren zu deren Herstellung.

**Stand der Technik**

Kosmetische Mittel zur Reinigung von Haar und Haut sollen nach heutigen Maßstäben eine Vielzahl an Anforderungen erfüllen. So soll neben der klassischen gewünschten Reinigungsleistung das Mittel den bei der Reinigung von Haut bzw. Haar verursachten Fett- und Wasserverlust so gering wie möglich halten, damit Weichheit und Glätte der Haare und/oder Haut möglichst erhalten bleiben. Häufig werden pflegende Zusatzstoffe in die Rezepturen einformuliert, um Glanz, Kämmbarkeit, Feuchtigkeit, Volumen oder weitere gewünschte Eigenschaften zu erzielen.

Aus der WO 2008/155075 sind kosmetische Zubereitungen beschrieben, die neben nicht-alkoxylierten Tensiden eine Mikroemulsion enthalten aus Alkylpolyglykosid, Glycerinmonoester, einem Ölkörper sowie Wasser. Diese kosmetischen Zubereitungen sind als Haarshampoos mit konditionierender Wirkung geeignet, da sie die Abscheidung von Silikonölen verbessern und verhältnismäßig hohe Mengen an Weichheit und Feuchtigkeit aufweisenden Ölen in Haarshampoos einbringen.

Aus der WO 2011/116881 wiederum sind kosmetische Reinigungsmittel bekannt, die Mikroemulsionen aus Alkyl(oligo)glykosid, Co-tensid, einer nicht-wasserlöslichen organischen Ölkomponente sowie ein Wachs und Wasser enthalten. Mit Hilfe dieser wachshaltigen Mikroemulsionen kann eine deutlich höhere Deposition von Lipidkomponenten auf der Haut erreicht werden, so dass die negativen Wirkungen von waschaktiven Substanzen wie Hautaustrocknung aufgehoben wird, ohne die reinigende Wirkung der Tenside zu verringern.

Eine zunehmende Anzahl von Verbrauchern möchte Produkte ohne Silikonöle, da diese eine Tendenz zu aufbauender Ablagerung auf dem Haar haben, weshalb die Haare schwer werden und kraftlos erscheinen. Weiterhin besteht ein Bedarf im Markt nach Produkten, die frei von kationischen Polymeren sind, aber dennoch eine gute Pflegeleistung aufweisen.

Die WO 2013/149853 offenbart haarkosmetische Produkte, insbesondere Conditioner, die neben pflanzlichen Lecithinen, anionische Tenside und Fettalkohole in einem Mengenverhältnis Lecithin : Aniontensid von 3:1 bis 1:1 enthalten. Neben den vielen guten Anwendungseigenschaften ist auch die native Herkunft vorteilhaft, wodurch es als natürliches, "grünes" Produkt zu betrachten ist.

Des Weiteren sind aus dem Internet Shampoos bekannt (XP055256722; URL:http://www.gnpd.com/sinatra/recordpage/1907709/fromsearch/kb7SQMQYz2/with_sort/ 2 sowie MINTEL@-Datenbank, 1. November 2014, Seiten 1-3, XP055204351; URL:http://www.gnpd.com/sinatra/recordpage/2785715/from searchDB12KUyFkX/), die unter anderem Lecithin, Alkylglucoside, Glycerinmonooleat und anionische Tenside enthalten und Triglyceride oder Distearylether als ölige Komponente.

In der US2013/012423 werden Mikroemulsionen für kosmetische Reinigungsmittel beschrieben, die ein Alkylglycosid, eine wasserunlösliche Ölkomponente und Wasser enthalten.

Aufbauend auf dem Konzept der "grünen" Produkte werden weitere Reinigungsmittel für Haut und Haar gesucht, die gute konditionierende Eigenschaften haben, jedoch ohne sich aufbauend auf Haut und Haar abzulagern.

Im Rahmen der vorliegenden Erfindung galt es, derartige "grüne" Reinigungsmittel für Haut und Haar bereitzustellen, die auf natürlichen Tensiden basieren, also insbesondere keine Tenside enthalten, die Ethylenoxid und/oder Propylenoxid aufweisen. Des Weiteren sollten die Reinigungsmittel vorzugsweise keine Silikone bzw. Silikonöle enthalten und bevorzugt auch frei von kationischen Polymeren sein.

Schließlich sollten die Reinigungsmittel leicht und möglichst einfach bei niedrigen Temperaturen herstellbar sein. Insbesondere sollte dem Hersteller ein konditionierend wirkendes Mittel an die Hand gegeben werden, das er anstelle seiner Silikone bzw. Silikonöle in seine eigene Basis-Reinigungsmittel-Formulierung bei niedrigen Temperaturen einarbeiten kann. Es sollte ein einfacher Austausch der Silikonverbindungen bzw. der kationischen Polymere in der Reinigungsmittelrezeptur ermöglicht werden, der keine negativen Auswirkungen auf Aussehen und Lagerstabilität der Reinigungsmittel hat. Weiterhin sollten die pflegenden Haarshampoos eine hohe Transparenz aufweisen, um den Kunden ein optisch ansprechendes Produkt anbieten zu können.

Die Aufgabe der vorliegenden Erfindung konnte gelöst werden durch die Verwendung von speziellen einphasigen, transparenten Tensid/Öl/Wasser-Zubereitungen enthaltend

(a) Alkyl- und/oder Alkenyloligoglykosid als Tensid

(b) Lecithin als Co-Tensid

(c) eine nicht-wasserlösliche organische Ölkomponente

(d) und Wasser.

Mikroemulsionen enthaltend nichtionische Zuckertenside, Lecithin und Fettsäureester sind wissenschaftlich untersucht worden.

So ist das Verhalten von Mikroemulsionen aus Lecithin und Isopropylmyristat als Öl unter Verwendung von Hexylpolyglucosid als hydrophilem Linker und Sorbitanmonooleat als lipophilem Linker in einem Artikel von E. Acosta et al. in Environ. Sci. Technol. 2005, 39, 1275-1282 beschrieben. Die Lecithin-basierten Mikroemulsionen werden als wasserbasierte Systeme als Ersatz für organische Lösungsmittel gesehen, die auch in kosmetischen Formulierungen verwendbar sind.

Untersuchungen zu Mikroemulsionen aus Lecithin, Decylpolyglucoside und Isopropylmyristat als Öl in Anwesenheit von kurzkettigen Alkoholen als Co-Tensid sind von F. Pattarino et al, J.Dispersion Science and Technology, 21(3), 345-263 (2000) durchgeführt wurden, wobei insbesondere die Rolle des kurzkettigen Alkohols als Co-Tensid im Mittelpunkt stand.

Mikroemulsionen, die ausschließlich auf natürlichen Tensiden basieren, werden von J. Schwarz et al. in European Journal of Pharmaceutics and Biopharmaceutics, 81 (2012), 557-562 diskutiert. Die Mikroemulsionen enthalten kurzkettige Alkohol als Co-Tensiden und Isopropylmyristat als Öl sowie Alkylpolyglucosid und Lecithin oder Lecithin und Sucroselaurat als Tensid.

## Gegenstand der Erfindung

Ein erster Gegenstand der Erfindung ist die Verwendung von einphasigen, transparenten Tensid/Öl/Wasser-Zubereitungen enthaltend

(a) Alkyl- und/oder Alkenyloligoglykosid als Tensid

(b) Lecithin als Co-Tensid

(c) eine nicht-wasserlösliche organische Ölkomponente

(d) und Wasser

als konditionierend wirkendes Mittel in Reinigungsmittel für Haut und Haar.

Ein weiterer Gegenstand der Erfindung sind ausgewählte einphasige, transparente Tensid/Öl/Wasser-Zubereitungen mit ausgewählten Mengen an Tensid, Co-Tensid, Öl und Wasser, vorzugsweise mit besonders effektiven Co-Tensid-Mischungen.

Ein dritter Gegenstand der vorliegenden Erfindung sind die kosmetischen milden Reinigungsmittel für Haut und Haar, die neben den konditionierend wirkenden Tensid/Öl/Wasser-Zubereitungen anionische Tenside sowie ggf. nichtionische Tenside und ggf. weitere kosmetische Zusatzstoffe und Wasser enthalten sowie ein Verfahren zu deren Herstellung.

Im Sinne der Erfindung sind als Reinigungsmittel für Haut und Haar kosmetische, reinigende Produkte zu verstehen wie Haarshampoos, Haarspülungen, Haarkuren, Duschbäder, Duschshampoos, Duschgele, Duschöle oder auch Kombinationen davon wie 2-1 Produkte, die gleichzeitig Körper- und Haarreinigung ermöglichen. Insbesondere richtet sich die vorliegende Erfindung auf Reinigungsmittel für das Haar, vorzugsweise Haarshampoos.

Im Sinne der vorliegenden Erfindung werden unter konditionierend wirkende Mittel auch solche Mittel verstanden, die nach der Behandlung auf der Haut ein angenehmes Hautgefühl hinterlassen, der durch direkten Kontakt der menschlichen Haut mit einem Stoff oder einer Stoffmischung ausgelöst wird. In der Praxis wird dieses Hautgefühl z.B. durch Panel-Tests an Probanden ermittelt, die ihre Sinneseindrücke in Bezug auf bestimmte Parameter, wie "Trockenheit der Haut", "Weichheit der Haut" etc. durch Benotungen qualifizieren. Auch diese verbesserten Eigenschaften werden im Sinne der Erfindung unter dem Begriff der konditionierenden Eigenschaften subsummiert.

Des Weiteren werden unter dem Begriff der konditionierenden Eigenschaften auch verbesserte Kämmbarkeiten der Haare nach Gebrauch des Reinigungsmittels verstanden, die auf eine glattere, weniger aufgeraute Oberflächenstruktur des Haares zurückzuführen ist. Die bessere Kämmbarkeit der Haare kann im nassen und/oder trockenen Zustand sowohl in den Längen als auch in den Spitzen (sog. Entwirrbarkeit) auftreten. Konditionierend wirkende Mittel zeigen weiterhin meist auf Haaren verbesserte taktile Eigenschaften wie Glätte, Weichheit, Geschmeidigkeit, Haarglanz, weniger elektrostatische Aufladung und bessere Frisierbarkeit.

Einphasig im Sinne der vorliegenden Erfindung sind Zubereitungen, die mit dem menschlichen Auge und im Lichtmikroskop als einheitliches und transparentes System wahrgenommen werden, d.h. die Größe der Ölstrukturen in Wasser sind so klein, dass sie nicht wahrgenommen werden. In der Regel ist die Größe der Ölstruktur (Öltröpfchen oder bikontinuierliche Strukturen) im Bereich von 1 bis 100 nm.

Besonders bevorzugt werden als einphasige Tensid/Öl/Wasser-Zubereitungen sogenannte Mikroemulsionen. Unter Mikroemulsionen werden zunächst alle makroskopisch homogenen, optisch transparenten, niedrigviskosen und insbesondere thermodynamisch stabile Mischungen aus zwei miteinander nicht mischbaren Flüssigkeiten und mindestens einem nichtionischen oder einem ionischen Tensid verstanden. Die mittleren Teilchengrößen der Mikroemulsionen liegen üblicherweise unter 100 nm, sie weisen eine hohe Transparenz auf und sind beim Zentrifugieren bei 2000 UPM für mindestens 30 Minuten gegenüber einer sichtbaren Phasenseparation stabil.

Die Transparenz wurde gemessen als FNU *Formazine Nephelometrie Units* - Streulichtmessung (Winkel 90°) gemäß den Vorschriften der Norm ISO 7027 mit Formazin als Trübungsstandardflüssigkeit. Im Sinne der Erfindung ist eine Zubereitung bis maximal 200 FNU gemessen nach der Norm als transparent zu bezeichnen.

Im Sinne der vorliegenden Erfindung werden als nicht-wasserlösliche Verbindungen solche Verbindungen verstanden, die in Wasser bei 20 °C eine Löslichkeit von kleiner gleich 2 Gew.- % aufweisen.

## Beschreibung der Erfindung

Überraschenderweise wurde nun gefunden, dass die erfindungsgemäß verwendeten einphasigen, transparenten Tensid/Öl/Wasser-Zubereitungen mindestens einen der folgenden Vorteile in den Reinigungsmitteln für Haut und Haar bewirken:

- leichtere Nasskämmbarkeit nach der Behandlung
- leichtere Kämmbarkeit im trockenen Zustand der Haare in Längen und Spitzen
- verbesserte taktile Eigenschaften wie Glätte, Weichheit und Geschmeidigkeit der Haare
- vermehrten Haarglanz
- geringere elektrostatische Aufladung
- reduzierter Splitt an den Haarspitzen
- Haut einen glatten, weichen Griff
- Reduzierung des spannenden Hautgefühls
- grünes Produkt, d.h. wirksame Bestandteile sind nativen Ursprungs
- keine Tenside mit Ethylenoxid und/oder Propylenoxid enthalten
- keine silikonhaltigen Verbindungen enthalten
- keine kationischen Polymere enthalten
- leichter Austausch der gängigen Silikonverbindungen durch die neuen Zubereitungen
- herstellbar bei niedrigen Temperaturen
- Farbe und Viskosität der Reinigungsmittel bleibt erhalten
- Lagerstabilität der Reinigungsmittel bleibt erhalten

Die Tensid/Öl/Wasser-Zubereitungen enthalten zwingend a) Alkyl- und/oder Alkenyloligoglykoside (im Folgenden auch als "APG" bezeichnet) als Tenside.

Alkyl- und/oder Alkenyloligoglucoside im Sinne der vorliegenden Lehre folgen vorzugsweise dabei der Formel (I)

$$R^1O\text{-}[G]p$$

in der $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und man hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad größer als 1,1 und kleiner als 1,7 ist und insbesondere zwischen 1,2 und 1,4 liegt.

Die Tensid/Öl/Wasser-Zubereitungen enthalten des Weiteren zwingend (b) Lecithin als Co-Tensid.

Im Sinne der vorliegenden Erfindung ist der Begriff Lecithin nicht auf Phosphatidylcholin beschränkt, sondern bezieht sich auf ein komplexes Stoffgemisch aus polaren, nicht in Aceton löslichen (Hauptbestandteil: Phospho- und Glycolipide), nichtpolaren, in Aceton löslichen Lipiden (Hauptbestandteil: Triglyceride) und weiteren Bestandteilen. Diese Definition entspricht der Beschreibung der EU für Lecithine im Nahrungsmittelbereich (E 322, vgl. Official Journal of the European Union L 253/39 vom 20.09.2008).

Im Sinne der vorliegenden Erfindung umfasst der Begriff Lecithin ebenfalls Lecithin, das mindestens einem Aufarbeitungsschritt unterzogen worden ist. Geeignete Aufarbeitungsschritte umfassen das Entfetten und/oder die Fraktionierung. Im Falle des Entfettens werden bzw. wird das Öl und/oder die freien Fettsäuren aus dem nativen Lecithin entfernt. Das Entfetten dient der Gewinnung von pulverförmigen oder granulierten sogenannten "reinen Lecithinen". Diese weisen eine im Vergleich mit nativen Lecithinen erhöhte Phospholipid-Konzentration auf sowie im Allgemeinen verbesserte Öl-in-Wasser-Emulgierbarkeit. Die Fraktionierung von Lecithin umfasst vorzugsweise eine einstufige oder mehrstufige Extraktion mit einem organischen Lösungsmittel oder einer Lösungsmittelmischung. Für die Extraktion wird vorzugsweise ein Alkohol oder ein Kohlenwasserstoff oder eine Kohlenwasserstoffmischung verwendet, insbesondere Ethanol oder Hexan. Die Fraktionierung des Lecithins umfasst sodann die Abtrennung in mindestens eine Fraktion, die in dem Auszugsmittel lösbar ist, und mindestens eine Fraktion, die in dem Auszugsmittel nicht lösbar ist. Anstelle von oder zusätzlich zu der extraktiven Fraktionierung ist es möglich, herkömmliche chromatographische Methoden zur Fraktionierung anzuwenden.

Im Sinne der vorliegenden Erfindung umfasst der Begriff Lecithin ebenfalls Lecithin, das mindestens einem chemischen Modifizierungsschritt unterzogen worden ist. Die Modifizierung ist vorzugsweise ausgewählt aus enzymatischer Hydrolyse, Acetylierung, Hydroxylierung, Hydrierung oder einer Kombination mindestens zweier der vorgenannten Verfahren. Die enzymatische Modifizierung beruht auf dem Abtrennen eines Fettsäuremoleküls von dem Phospholipidmolekül mit Hilfe einer Phospholipase. Die Acetylierung ist eine Modifizierung des in dem Lecithin vorliegenden Phosphatidylethanolamin durch Acylierung der Aminogruppe mit einem Acetylrest. Die Hydroxylierung der ein- und mehrfach ungesättigten Fettsäuren, die in dem Phospholipidmolekül gebunden sind, kann beispielsweise durch Reaktion mit Wasserstoffperoxid stattfinden. Die Hydrierung der ein- und mehrfach ungesättigten Fettsäuren, die in dem Phospholipidmolekül gebunden sind, kann beispielsweise durch Reaktion mit Wasserstoff in Gegenwart von herkömmlichen Hydrierkatalysatoren stattfinden. Das übliche im Handel erhältliche Lecithin ist eine Zusammensetzung, die hauptsächlich Phospholipide sowie Glycolipide, Triglyceride und begleitende Stoffe (wie Sterine, freie Fettsäuren, Tocopherole, Phenolsäuren, Sinapin usw.), und ebenfalls kleine Mengen an Kohlenhydraten umfasst.

Vorzugsweise weist das als Bestandteil b) in der Erfindung verwendete Lecithin einen Gesamtgehalt an Phospholipiden von mindestens 35 Gew.-%, besonders bevorzugt mindestens 38 Gew.-%, insbesondere mindestens 40 Gew.-%, bezogen auf das Gesamtgewicht von Bestandteil b) auf.

Bevorzugt weist das als Bestandteil b) in der Erfindung verwendete Lecithin einen Gehalt an neutralen Lipiden (d.h. in Aceton lösliche Lipide) von mindestens 20 Gew.-%, besonders bevorzugt mindestens 25 Gew.-%, bezogen auf das Gesamtgewicht von Bestandteil b) auf.

Geeignet hat das als Bestandteil b) verwendete Lecithin eine Iodzahl von mindestens 10f. Die Iodzahl ist ein Maß für den Gehalt an ungesättigten Bestandteilen in dem Lecithinbestandteil b) und bezieht sich auf die Menge an Iod in Gramm, die formal zu 100 Gramm an Lecithinbestandteil b) hinzugerechnet werden kann.

Vorzugsweise weist das als Bestandteil b) in der Erfindung verwendete Lecithin nach Spaltung einen Gehalt an Ölsäuren von mindestens 35 Gew.-%, besonders bevorzugt von mindestens 40 Gew.-%, bezogen auf das Gesamtgewicht von Bestandteil b) auf. Der Ölsäuregehalt bezieht sich hier auf Bestandteil b) nach dem Abtrennen eines Fettsäuremoleküls von dem Phospholipidmolekül. Dies findet beispielsweise mittels enzymatischer Hydrolyse statt, z.B. mit Hilfe von Phospholipase A2.

Vorteilhafterweise weist das als Bestandteil b) in der Erfindung verwendete Lecithin ein qualitatives Gewichtsverhältnis von Phosphatidylcholin und Phosphatidylethanolamin von $\geq 1$ auf.

Als Bestandteil b) geeignete pflanzliche Lecithine sind ausgewählt aus Rapslecithin, Sojalecithin, Maislecithin, Sonnenblumenlecithin und/oder deren Mischungen.

In einer speziellen Ausführungsform umfasst Bestandteil b) kein Lecithin aus genetisch veränderten Pflanzen. Diese Maßgabe kann vorzugsweise durch die Verwendung von Rapslecithin erfüllt werden, da aktuell zumindest in der EU keine genetisch veränderten Rapspflanzen zur Kultivierung zugelassen sind.

Vorzugsweise besteht Bestandteil b) zu mindestens 30 Gew.-%, besonders bevorzugt zu mindestens 50 Gew.-%, insbesondere zu mindestens 75 Gew.-%, speziell zu mindestens 90 Gew.-% aus Rapslecithin, bezogen auf das Gesamtgewicht von Bestandteil b). In einer speziellen Ausführungsform wird ausschließlich Rapslecithin als Bestandteil b) verwendet. Der Begriff "Raps" bezieht sich allgemein auf Ölsaaten, die von der Pflanzengattung *Brassica* (Kohl) stammen. Prinzipiell geeignet zum Erhalten von Rapslecithin sind die verschiedenen Rapskulturen, die weltweit verfügbar sind, z.B. die Pflanzensorten *Brassica carinata, Brassica juncea, Brassica rapa* und *Brassica napus.* Eine speziell in Mitteleuropa kultivierte Rapssorte ist der Winterraps (*Brassica napus* L).

Eine umfassende Beschreibung der Zusammensetzung und Wirkungsweise von Lecithin aus Raps ist in der Dissertation von Claudia Heift, Institut für Biochemie und Lebensmittelchemie, Universität Hamburg, 2007 zu finden.

Geeignetes Lecithin weist beispielsweise die folgende Zusammensetzung auf (in Gew.-%) :

Triglyceride: 18 - 42 %

Gesamtmenge an Phospholipiden: 38 - 52 %
Phosphatidylcholin: 13 - 17 %, vorzugsweise 14 - 16 %
Phosphatidylethanolamin: 6 - 10 %
Phosphatidylinositol: 9,0 - 11 %.
Phosphatidsäure: 2 - 6 %
Gesamtmenge an Glycolipiden: 6 - 10 %
Sterylglycoside: 4 - 6 %
Digalactosyldiacylglyceride: 2 - 4 %
Cerebroside: 0,5 - 1,5 %
Kohlenhydrate: 3 - 12 %
Gesamtmenge an begleitenden Stoffen: 0,5 - 5 %
$\alpha$-Tocopherol: 10-2500 ppm
Sterine, Phenolsäure (Sinapin), Chlorophyll, usw.

Das als Bestandteil b) in der Erfindung verwendete Rapslecithin weist vorzugsweise einen Triglyceridanteil im Bereich von 32 Gew.-% bis 42 Gew.-% auf. Ein wesentlicher Unterschied zwischen Rapslecithin einerseits und Soja- oder Sonnenblumenlecithin andererseits liegt in der Zusammensetzung der Fettsäuren. Beispielsweise weist Rapslecithin einen höheren Anteil an Ölsäure und einen niedrigeren Anteil an Linolsäure auf.

Besonders bevorzugt wird Rapslecithin mit einem Triglyceridanteil im Bereich von 32 Gew.-% bis 42 Gew.-% und einem Gehalt an - bezogen auf den Gesamtgehalt an Fettsäure im Triglycerid:

Palmsäure: 4 - 8 Gew.-%
Stearinsäure: 0,5 - 3 Gew.-%
Ölsäure: 35 - 45 Gew.-%
Linolsäure: 12 - 22 Gew.-%
Linolensäure: 2 - 5 Gew.-%.

Ein im Handel erhältliches zur Verwendung als Bestandteil b) geeignetes Rapslecithin ist Solec RF-10 von Solae Europe S.A., Ouderkerke a.d. ljssel, NL.

Im Sinne der vorliegenden Erfindung entspricht es einer Ausführungsform der vorliegenden Erfindung, wenn neben den Lecithinen zusätzlich ein Co-Tensid (b2) enthalten ist ausgewählt aus der Gruppe, die gebildet wird von Glycerinmonofettsäureester, Partialester von Polyglycerin mit durchschnittlichen Eigenkondensationsgraden von 2 bis 8, Partialester von Pentaerythrit und Partialester von Trimethylolpropan.

Als Partialester von Polyglycerin mit durchschnittlichen Eigenkondensationsgraden von 2 bis 8 sind vorzugsweise die Partialester von C12 bis C22-Fettsäuren, bevorzugt die Partialester der Hydroxyfettsäuren mit 12 bis 22 Kohlenstoffatomen und insbesondere der Dihydroxystearinsäureester des Polyglycerins zu nennen, der unter dem Handelsnamen Dehymuls® PGPH von der BASF vertrieben wird.

Bevorzugt handelt es sich bei dem Co-Tensid (b2) um einen Monoester einer Fettsäure der Kettenlänge C12-C22 mit Glycerin, wobei insbesondere Monoester von Glycerin mit ungesättigten linearen Fettsäuren geeignet sind. Besonders bevorzugt im Sinne der Erfindung (b2) Glycerinmonooleat ist. Im Sinne der Erfindung sind sowohl das reine Glycerinmonooleat als auch deren technische Qualitäten geeignet. Ein geeignetes technisches Produkt ist beispielsweise Monomuls 90-O 18®, ein Handelsprodukt der BASF Personal Care & Nutrition GmbH.

Des Weiteren enthalten die Zubereitungen als zwingenden Bestandteil c) eine organische, nicht-wasserlösliche Ölkomponente. Es handelt sich hierbei um bei Raumtemperatur flüssige Verbindungen (sogenannte Öle), das bedeutet die Verbindungen haben einen Schmelzpunkt unter bzw. einen Siedepunkt über den üblichen Raumtemperaturen von ca. 15 bis 25 °C.

Vorzugsweise weisen die organischen, nicht-wasserlöslichen Verbindungen eine Polarität zwischen 5 und 60 mN/m auf. Die Grenzflächenspannung wird bestimmt als Grenzflächenspannung in mN/m in Analogie der ASTM-Methode D971-99a (2004).

Geeignete Verbindungen c) sind beispielsweise ausgewählt aus der Gruppe, die gebildet wird von Guerbetalkoholen auf Basis von Fettalkoholen mit 6 bis 18 C-Atomen, Estern linearer $C_6$-$C_{22}$-Fettsäuren mit linearen $C_6$-$C_{22}$-Fettalkoholen, Triglyceriden auf Basis $C_6$-C14-Fettsäuren, Estern von $C_2$-$C_{12}$-Dicarbonsäuren mit linearen Alkoholen mit 1 bis 22 Kohlenstoffatomen oder Polyolen mit 2 bis 10 Kohlenstoffatomen und 2 bis 6 Hydroxylgruppen, pflanzlichen Ölen, primären Alkoholen, linearen $C_6$-$C_{22}$-Dialkylcarbonaten, Guerbetcarbonaten, linearen, und symmetrischen oder unsymmetrischen Dialkylethern mit 6 bis 22 Kohlenstoffatomen pro Alkylgruppe.

Beispiele für die Gruppe der Ester von Fettsäuren oder Fettalkoholen sind die sogenannten Esteröle wie Isopropylpalmitat, Isopropylmyristat, Ethylhexylpalmitat, Ethylhexylstearate, Di-n-Octylcarbonate, Caprylylcaprylat, Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat oder deren Mischungen. Dioctyl Malat ist ein Beispiel für einen geeigneten Dicarbonsäureester.

Als Triglyceride eignen sich insbesondere Glycerinester von Fettsäuren mit 8 und/oder 10 Kohlenstoffatome, wobei die Triglyceridester auch Anteile an Diglyceridester enthalten können, beispielsweise Triglycerid-/Diglyceridester wie sie erhältlich sind unter dem Handelsnamen Myritol® 312 von der BASF Personal Care & Nutrition GmbH (INCI Name: Caprylic/Capric triglyceride).

Geeignet sind des weiteren pflanzlichen Öle wie Erdnussöl, Rizinusöl, Kokosnussöl, Maisöl, Olivenöl, Palmkernöl, Sonnenblumenöl, Sojaöl, Rapsöl, Mandelöl, Traubenkernöl, Distelöl, Weizenkeimöl, Nachtkerzenöl, Macadamianussöl, Arganöl und/oder Avocadoöl.

Bevorzugt sind im Sinne der Erfindung als c) verzweigte Fettalkohole, C6-C22 Dialkylether, Esteröle, Kohlenwasserstoffe und C6-C22 Dialkylcarbonate.

Insbesondere geeignet sind als organische Ölkomponente (c) lineare C6-C12-Dialkylether und/oder lineare C6-C22-Fettalkoholdicarbonate oder deren Mischungen.

Ganz besonders bevorzugt sind als nicht-wasserlösliche organische Ölkomponente (c) Dicaprylether wie Cetiol® OE, ein Handelsprodukt der BASF Personal Care & Nutrition GmbH, und/oder Dicaprylcarbonat wie Cetiol® CC, ein Handelsprodukt der BASF Personal Care & Nutrition GmbH.

Im Sinne der Erfindung sind unter Dicaprylether bzw. Dicaprylcarbonat sowohl die reinen Verbindungen auf Basis von ausschließlich C8- Fettalkoholen als auch solche Verbindungen zu verstehen, die aus technischen Mischungen von C8- Fettalkoholschnitten hergestellt werden und untergeordnete Mengen an C6/C10/C12 Kohlenstoffatomen aufweisen.

Im Sinne der Erfindung ist die Ölkomponente c) verschieden von b2).

Als weitere fakultative Inhaltsstoffe können in den Zubereitungen solche enthalten sein, die ausgewählt sind aus der von Hydrotope, Konservierungsmittel, Antioxidantien, biogene Wirkstoffe, Biozide und pH-Regulantien gebildeten Gruppe, beispielsweise Glycerin, Zitronensäure, Benzoesäure und/oder Phenoxyethanol. Geeignete Vertreter derartiger Verbindungen sind in der folgenden Beschreiung der fertigen Reinigungsmittel als Komponente D) offenbart.

Schließlich enthalten die erfindungsgemäß verwendeten Zubereitungen noch Wasser als zwingenden Bestandteil d) auf.

Im Allgemeinen enthalten die erfindungsgemäß verwendeten Zubereitungen in Mengen von

5 bis 25 Gew.-%, vorzugsweise 5 bis 20 Gew.-% der Komponente (a)
5 bis 25 Gew.-%, vorzugsweise 7,5 bis 20 Gew.-% der Komponente (b)
0 bis 15 Gew.-% der Komponente (b2)
5 bis 50 Gew.-%, vorzugsweise 15 bis 30 Gew.-% der Komponente (c) und
0 bis 10 Gew.-% weitere fakultative Inhaltsstoffe sowie
ad 100 Gew.-% Wasser als Komponente (d) - Gew.-% bezogen auf die verwendete Zubereitung.

Sofern die erfindungsgemäß verwendeten Zubereitungen ausschließlich Lecithin als Co-Tensid (b) enthalten, empfehlen sich folgende Mengen:

5 bis 15 Gew.-%, vorzugsweise 7,5 bis 12,5, Komponente (a)
10 bis 25 Gew.-%, vorzugsweise 15 bis 20 Gew.-%, Komponente (b)
15 bis 25 Gew Komponente (c) und
0 bis 10 Gew.-% weitere Inhaltsstoffe sowie
ad 100 Gew.-% Wasser als Komponente (d) enthalten - Gew.-% bezogen auf die verwendete Zubereitung.

Bevorzugt enthalten die erfindungsgemäß verwendeten Zubereitungen Lecithin als Co-Tensid b) und zusätzlich eines der oben beschriebenen Co-Tenside (b2). Unter diesen Bedingungen empfehlen sich in den erfindungsgemäß verwendeten Zubereitungen folgende Mengen zu verwenden:

10 bis 20 Gew .-%, vorzugsweise 12,5 bis 17,5 Gew.-% der Komponente (a)
5 bis 15 Gew.-%, vorzugsweise 7,5 bis 12,5 Gew.-% der Komponente (b)
5 bis 15 Gew.-%, vorzugsweise 7,5 bis 12,5 Gew.-% der Komponente (b2)
15 bis 25 Gew.-% der Komponente (c) und

0 bis 10 Gew.-% weitere Inhaltsstoffe sowie
ad 100 Gew.-% Wasser als Komponente (d) - Gew.-% bezogen auf die verwendete Zubereitung.

Insbesondere geeignet sind erfindungsgemäß verwendete Zubereitungen aus:

12,5 bis 17,5 Gew.-% von Alkyloligoglukoside der Formel (I), wobei R1 für eine Mischung von Alkylresten mit 6 bis 12 Kohlenstoffatome steht,
7,5 bis 12,5 Gew.-% Rapslecithin als Komponente (b)
7,5 bis 12,5 Gew.-% Glycerinmonooleat als Komponente (b2)
15 bis 25 Gew.-% Dicaprylylether als Komponente (c)
0 bis 2 Gew.-% weitere Inhaltsstoffe, vorzugsweise pH-Regulantien und Konservierungsmittel und ad 100 Gew.-% Wasser, wobei Gew.-% bezogen auf Zubereitung ist.

Die erfindungsgemäß verwendeten Zubereitungen weisen vorzugsweise einen pH-Wert zwischen 3 und 7 auf, wobei die Bereiche von 3,5 bis 5,5 vorteilhaft sind.

Die Herstellung der erfindungsgemäß verwendeten Zubereitungen erfolgt durch einfaches Vermischen und Erwärmen über den Schmelzpunkt aller Komponenten, beispielsweise im einstufigen Verfahren gemäß WO08/155075 A1, wobei zu einer vorgelegten wässrigen Lösung der Alkyl- und/oder Alkenylglykoside (Komponente a) die anderen zwingenden sowie fakultativen Komponenten eingerührt, vorzugsweise bei 25 bis 80 °C und ggf. unter moderatem Rühren.

Es bilden sich einphasige, transparente Zubereitungen aus, die thermodynamisch stabil sind. Die erfindungsgemäß verwendeten Zubereitungen wirken als konditionierendes Mittel und verbessern die konditionierenden Eigenschaften der kosmetischen Reinigungsmittel, vorzugsweise die konditionierenden Eigenschaften von Haarshampoos. Insbesondere tragen sie hier zu der Verbesserung der Kämmbarkeit, der Glätte, der Geschmeidigkeit und des Glanzes der menschlichen Haare bei.

Die erfindungsgemäß verwendeten Zubereitungen werden vorzugsweise als Vorformulierung den kosmetischen Reinigungsmitteln zugegeben. Damit ist ein Austausch von anderen konditionierend wirkenden Mitteln in Reinigungsmitteln problemlos möglich.

Falls gewünscht können die erfindungsgemäß verwendeten Zubereitungen jedoch auch in verdünnter Form oder durch Verdicker mit höherer Viskosität verwendet werden.

Ein weiterer Gegenstand betrifft die einphasigen, transparenten Tensid/Öl/Wasser-Zubereitungen für Reinigungsmittel für Haut und Haar, dadurch gekennzeichnet, dass sie in Mengen von

(a) 5 bis 25 Gew.-%, vorzugsweise 5 bis 20 Gew.-% Alkyl- und/oder Alkenyloligoglykosid als Tensid
b) 5 bis 25 Gew.-%, vorzugsweise 7,5 bis 20 Gew.-% Lecithin als Co-Tensid
b2) bis 15 Gew.-% ein weiteres Co-Tensid ausgewählt aus der Gruppe, die gebildet wird von Glycerinmonofettsäureester, Partialester von Polyglycerin mit durchschnittlichen Eigenkondensationsgraden von 2 bis 8, Partialester von Pentaerythrit und Partialester von Trimethylolpropan
c) 5 bis 50 Gew.-%, vorzugsweise 15 bis 30 Gew.-% einer nicht-wasserlöslichen organischen Ölkomponente und
0 bis 10 Gew.-% weiterer Inhaltsstoffe sowie
d) ad 100 Gew.-% Wasser als Komponente d) - Gew.-% bezogen auf Zubereitung - enthalten.

Schließlich betrifft ein weiterer Gegenstand der vorliegenden Erfindung kosmetische Reinigungsmittel für Haut und Haar enthaltend milde kosmetische Reinigungsmittel für Haut und Haar enthaltend

A) konditionierend wirkende einphasige, transparente Tensid/Öl/Wasser-Zubereitungen in Mengen von
a) 5 bis 25 Gew.-%, vorzugsweise 5 bis 20 Gew.-% Alkyl- und/oder Alkenyloligoglykosid als Tensid
b) 5 bis 25 Gew.-%, vorzugsweise 7,5 bis 20 Gew.-% Lecithin als Co-Tensid
b2) 0 bis 15 Gew.-% ein weiteres Co-Tensid ausgewählt aus der Gruppe, die gebildet wird von Glycerinmonofettsäureester, Partialester von Polyglycerin mit durchschnittlichen Eigenkondensationsgraden von 2 bis 8, Partialester von Pentaerythrit und Partialester von Trimethylolpropan
c) 5 bis 50 Gew.-%, vorzugsweise 15 bis 30 Gew.-% eine nicht-wasserlösliche organischen Ölkomponente und
0 bis 10 Gew.-% weiterer Inhaltsstoffe sowie
d) ad 100 Gew.-% Wasser als Komponente d) - Gew.-% bezogen auf Zubereitung
B) anionische Tenside sowie
C) ggf. nichtionische, zwitterionische und/oder amphotere Tenside und
D) ggf. weitere kosmetische Zusatzstoffe und/oder Wasser.

Im Sinne der vorliegenden Erfindung enthalten die erfindungsgemäßen kosmetischen Mittel neben den schon beschriebenen einphasigen, transparenten Zubereitungen nach Patentanspruch 14 zwingend die anionische Tenside B).

Vorzugsweise sind die anionischen Tenside B) ausgewählt aus der Gruppe, die gebildet wird von Seifen, Alkylbenzolsulfonate, Alkansulfonate, Olefinsulfonate, $\alpha$-Methylestersulfonate, Sulfofettsäuren, Alkylsulfate, Hydroxy-mischethersulate, Monoglyceridsulfate, Fettsäureamidsulfate, Mono- und Dialkylsulfosuccinate, Mono- und Dialkylsulfosuccinamate, Sulfotriglyceride, Amidseifen, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkyloligoglucosidcarboxylate, Proteinfettsäurekondensate (insbesondere pflanzliche Produkte auf Weizenbasis) und Alkylphosphate; besonders bevorzugt sind Alkylsulfate alleine oder in Mischung mit weiteren milden anionischen Tensiden wie Acylglutamate und/oder Alkyloligoglucosidcarboxylate.

Alkylsulfate ("Fettalkoholsulfate") stellen bekannte anionische Tenside dar, die großtechnisch durch $SO_3$- oder Chlorsulfonsäure (CSA)-Sulfatierung von Fettalkohol- und nachfolgende Neutralisation hergestellt werden. Im Sinne der Erfindung kommen Sulfate in Betracht, die der Formel

$$R^2O\text{-}SO_3X$$

folgen, in der $R^2$ für einen linearen oder verzweigten Alkyl- und/oder Alkenylrest mit 6 bis 22 Kohlenstoffatomen und X für ein Alkali- und/oder Erdalkalimetall, Ammonium, Alkylammonium, Alkanolammonium oder Glucammonium steht. Typische Beispiele sind die Sulfate Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze.

Besonders bevorzugt ist das Natriumsalz des Sulfats auf Basis des Alkohols abgeleitet von der Kokosfettsäure.

Die anionischen Tenside B) sind vorzugsweise in Mengen von 5 bis 50 Gew.-%, bevorzugt 5 bis 25 und insbesondere 8 bis 20 Gew.-% - bezogen auf kosmetisches Reinigungsmittel - enthalten.

Weitere Tenside C)

Falls gewünscht können die kosmetischen Reinigungsmittel zusätzlich nichtionische Tenside, und/oder amphotere bzw. zwitterionische Tenside enthalten.

Bevorzugt sind von den weiteren nichtionischen Tensiden C) Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Bevorzugt sind nichtionische Tenside ohne Ethylenoxid- und/oder Propylenoxid-Einheiten enthalten.

Typische Beispiele für amphotere bzw. zwitterionische Tenside als weitere Tenside C) sind Alkylbetaine, Alkylamidobetaine, Aminopropionate, Aminoglycinate, Imidazoliniumbetaine und Sulfobetaine, besonders bevorzugt ist Cocamidopropylbetain. Bei den genannten Tensiden handelt es sich ausschließlich um bekannte Verbindungen.

Typische Beispiele für besonders geeignete milde, d.h. besonders hautverträgliche Tenside sind Monoglyceridsulfate, Mono- und/oder Dialkylsulfosuccinate, Fettsäureisothionate, Fettsäuresarcosinate, Fettsäuretauride, Fettsäureglutamate, $\alpha$-Olefinsulfonate, Alkyloligoglucoside, Fettsäureglucamide, Alkylamidobetaine wie Cocamidopropylbetain, Amphoacetate wie Sodiumcocoamphoacetat und/oder Proteinfettsäurekondensate, letztere vorzugsweise auf Basis von Weizenproteinen.

Besonders bevorzugt sind als amphotere bzw. zwitterionische Tenside Amphoacetate enthalten.

Die Tenside C) sind optional, wenn auch bevorzugt in Mengen von 0,2 bis 20 Gew.-%, insbesondere bevorzugt von 5 bis 15 Gew.-% enthalten - bezogen auf kosmetisches Mittel.

Weitere kosmetische Zusatzstoffe D)

Falls gewünscht können die kosmetischen Reinigungsmittel noch weitere typische Zusatzstoffe enthalten, die dem Fachmann bekannt sind, wie beispielsweise Emulgatoren, Perlglanzwachse, Stabilisatoren, Salz, Verdickungsmittel, Konsistenzgeber, Selbstbräuner, Pigmente, Antioxidationsmittel, Antischuppenmittel, Filmbildner, Quellmittel, Insektenrepellentien, Deodorant- und Antitranspirantwirkstoffe, biogene Wirkstoffe, Feuchthaltemittel (z.B. Glycerin, Sorbitol). Als biogene Wirkstoffe sind dabei insbesondere Tocopherol, Tocopherolacetat, Tocopherolpalmitat, Desoxyribonucleinsäure, Coenzym Q10, Ascorbinsäure, Retinol- und Retinylderivate, Bisabolol, Allantoin, Phytantriol, Panthenol, AHA-Säuren, Aminosäuren, Ceramide, essentielle Öle, Hyaluronsäure, Creatin, Proteinhydrolysate, Pflanzenextrakte, Pep-

tide und Vitaminkomplexe bevorzugt. Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate und hydrophob modifizierte Polyacrylate, Polyacrylamide, Polymere, Polyvinylalkohol und Polyvinylpyrrolidon. Als besonders wirkungsvoll haben sich auch Bentonite erwiesen, bei dem es sich um eine Mischung aus Cyclopentasiloxan, Disteardimonium Hectorit und Propylencarbonat handelt. Weiter in Frage kommen Ester von Fettsäuren mit Polyolen wie beispielsweise Pentaerythrit oder Trimethylolpropan wie Arlypon TT®, ein Handelsprodukt der BASF Personal Care and Nutrition GmbH, sowie Elektrolyte wie Kochsalz und Ammoniumchlorid. Besonders bevorzugt werden als Verdicker Mischungen des Xanthan-Gums.

Falls Konservierungsmittel erfindungsgemäß eingesetzt werden, sind diese bevorzugt ausgewählt aus der Gruppe, die gebildet wird von Benzoesäure und deren Salze, Zitronensäure und deren Salze, Phenoxyethanol, Benzylalkohol, Alkylparabenen, bevorzugt Ethyl-, Methyl- und Propylparaben. Als Konservierungsmittel eignen sich weiterhin beispielsweise Formaldehydlösung, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

## Herstellung der kosmetischen Reinigungsmittel

Die Herstellung der kosmetischen Reinigungsmittel erfolgt vorzugsweise derart, dass die Tensid/Öl/Wasser-Zubereitungen A) zu den vorgelegten weiteren Inhaltsstoffen der erfindungsgemäßen kosmetischen Reinigungsmitteln B) sowie ggf. C) und D) und ggf. Wasser eingerührt wird. Zur Herstellung von klaren Produkten empfiehlt sich die Zugabe der Tensid/Öl/Wasser-Zubereitung A) zu den weiteren Bestandteilen des Reinigungsmittels bei Raumtemperatur (Kaltherstellung) oder bei leicht erhöhten Temperaturen, vorzugsweise bei 30 bis 60 °C.

Eine Kaltherstellung ist nur aufgrund der vorformulierten Tensid/Öl/Wasser-Zubereitungen A) möglich. Daher betrifft ein weiterer Gegenstand der vorliegenden Erfindung ein Verfahren zur Herstellung von milden kosmetischen Reinigungsmitteln für Haut und Haar, dadurch gekennzeichnet, dass die transparente Tensid/Öl/Wasser-Zubereitung A) in die vorgelegten weiteren Inhaltsstoffe B) sowie ggf. C) und D) und ggf. Wasser bei Raumtemperatur eingerührt wird.

In einer bevorzugten Ausführungsform enthalten die erfindungsgemäßen kosmetischen Reinigungsmittel

A) 0,1 bis 10 Gew.-% einer erfindungsgemäß zu verwendenden Tensid/Öl/Wasser-Zubereitungen
B) 5 bis 40 Gew.-% anionische Tenside, vorzugsweise Fettalkoholsulfate
C) 0 bis 20 Gew.-% weitere nichtionische und/oder amphotere und/oder kationische Tenside
D) 0 bis 10 Gew.-% kosmetische Zusatzstoffe

sowie ggf. ad 100 Gew.-% weiteres Wasser.

Besonders bevorzugt ist ein kosmetisches Reinigungsmittel enthaltend

A) 1 bis 5 Gew.-% einer erfindungsgemäß zu verwendenden Tensid/Öl/Wasser-Zubereitungen
B) 5 bis 15 Gew.-% anionische Tenside, vorzugsweise Fettalkoholsulfate
C) 0,5 bis 10 Gew.-% weitere nichtionische und/oder amphotere und/oder kationische Tenside
D) 0,1 bis 5 Gew.-% kosmetische Zusatzstoffe

sowie ggf. ad 100 Gew.-% weiteres Wasser.

Die erfindungsgemäßen kosmetischen Reinigungsmittel können als feinteilige Emulsionen vorliegen wie bei Duschgelen, Duschbäder, Haarshampoos, Haarlotionen, Schaumbäder, Gesichtsreinigern, Make-up-Entfernern, Badezubereitungen, Babypflegeprodukten, als Cremes, als Gele, als Lotionen, als alkoholische und wässerig/alkoholische Lösungen, Emulsionen, Wachs/Fett-Massen, Stiftpräparaten, Pudern oder Salben.

Die kosmetischen Reinigungsmittel können auch als Tränkungsmedium für Tücher, Gewebe dienen, die nass oder trocken vom Verbraucher angewendet werden oder auch aus einem Pumpfoamer appliziert werden.

## Beispiele

Die Mikroemulsionen M1 und M2 wurden wie folgt hergestellt: die Alkylglucoside des Types Plantacare® wurden mit Benzoesäure und Wasser bei 40 °C erhitzt, dann auf 70 °C erhitzt. Nach Zugabe der weiteren Bestandteile wurde unter moderater Mechanik (Flügelrührer mit 850 rpm) bei 75°C gerührt, bis eine homogene Zubereitung vorlag (maximal eine Stunde). Unter Rühren wurde auf 30 °C abgekühlt und der pH-Wert mit Zitronensäure eingestellt.

Die Mengenangabe "AS" ist der Aktivsubstanzgehalt (Wirkstoffgehalt), angegeben in % und bezogen auf die gesamte Mikroemulsion

Tabelle 1: Mikroemulsionen

| Inhaltsstoffe | M1 | M2 |
|---|---|---|
| Rapslecithin | 8,93 | 18,00 |
| Glycerylmonooleat | 8,93 | - |
| Dicaprylylether | 17,86 | 20,00 |
| Plantacare 810 UP® ; C8-C10-Fettalkohol-1.4-glucosid; INCI:Caprylyl/ Capryl Glucoside | 9,43 | - |
| Plantacare 1200 UP ® ;C10-C16-Fettalkohol-1.4-glucosid; INCI: Laurylglucoside | 6,83 | - |
| Plantacare 2000 UP ® ;C10-Fettalkohol-1.4-glucosid; INCI: Decylglucoside | - | 9,50 |
| Benzoesäure | 0,45 | 0,50 |
| Zitronensäure (50%ig) | qs pH 4,0 | qs pH 4,0 |
| Wasser, dest. | ad 100 Gew.-% | ad 100 Gew.-% |
| Mikroemulsion Aussehen | transparent, einphasig | transparent, einphasig |

Die Transparenz des Konzentrates M1 wurde bestimmt nach der Methode der turbidischen Verhältnismessung zwischen einem primären nephelometrischen Streulicht (90°) und einem Transmissionssignal mit dem Trübungsmessgerät 2100 Qis, Hach Lange GmbH (Lichtquelle Leuchtdiode (LED) mit 860 nm; Detektor: Silizium-Photodiode; Messbereich 0 - 1000 FNU (FNU). Ergebnis: 136 FNU

2. Rezepturen für Haarshampoos mit Mikroemulsionen ohne kationisches Polymer

Die nachfolgenden Rezepturen sollen die vorliegende Erfindung verdeutlichen, ohne sie einzuschränken. Die Mengenangabe "AS" ist der tatsächliche Aktivsubstanzgehalt (Wirkstoffgehalt), angegeben in % und bezogen auf das gesamte Haarshampoo. AS wird berechnet durch Multiplikation der Menge mit dem Substanzgehalt der einzelnen Komponenten.

Die Mikroemulsionen M1 bzw. M2 werden als ein wirksamer Bestandteil betrachtet, dem der Substanzgehalt von 100 zugeordnet ist, so dass hier der AS der tatsächlich eingewogenen Menge entspricht, angegeben in %.

Alle mit® gekennzeichneten Substanzen sind eingetragene Marken.

Die Haarshampoos wurden hergestellt durch Verrühren der Bestandteile bei etwa 50°C. Bei Anwesenheit des Verdickers Rheocare® XG (= Xanthan) empfiehlt es sich, Xanthan mit der Hälfte der Wassermenge aufquellen zu lassen, die restlichen Tenside (Plantapon® SF, Sulfopon® 1216 G und/oder Plantacare® 810) mit der restlichen Wassermenge zu homogenisieren und anschließend diese Tensidlösung zu dem gequollenen Verdicker zu geben. Zum Schluss wurde die vorformulierte Mikroemulsion zugegeben und der pH-Wert eingestellt. Sofern kein Verdicker enthalten ist, wurde die gesamte Wassermenge zur Herstellung der Tensidlösung eingesetzt, die Tensidlösung mit der vorformulierten Mikroemulsion versetzt und der pH-Wert eingestellt.

Die erfindungsgemäßen Haarshampoos sind H1, H2, H3 und H4; um die konditionierende Wirkung an sich zu zeigen, wurde ihnen als Vergleich die Haarshampoos V1, V3 und V4 gegenüber gestellt (siehe Tabelle 2).

Nur mit den vorformulierten einphasigen Tensid/Öl/Wasser-Zubereitungen M1 und M2 sind klare Haarshampoo-Formulierungen möglich.

Wenn die Bestandteile von M1 und M2 einzeln, also nicht in Form einer einphasigen Zubereitung in die Haarshampoo-Formulierungen bei RT eingerührt werden, werden keine stabilen, klaren Haarshampoos erhalten (siehe Tabelle 3, V5 - V7).

Tabelle 2: Haarshampoo

| Ingredient | INCI | V1 [% AS] | H1 [% AS] | H2 [% AS] | V3 [% AS] | H3 [% AS] | V4 [% AS] | H4 [% AS] |
|---|---|---|---|---|---|---|---|---|
| Plantapon SF® | Sodium Cocoamphoacetate Glycerin Lauryl Glucoside Sodium Cocoyl Glutamate Sodium Lauryl Glucose Carboxylate | 16,8 | 16,8 | 16,8 | - | - | - | - |
| Sulfopon 1216G® | Sodium Coco-Sulfate | - | - | - | 10,0 | 10,0 | 9,0 | 9,0 |
| Plantacare 810 UF® | Capryl/Capryl Glucoside | - | - | - | - | - | 3,0 | 3,0 |
| Mikroemulsion M1 | Lecithin-Mikroem. gem. Tabelle 1 | 0,0 | - | 4,0 | 0,0 | 4,0 | 0,0 | 4,0 |
| Mikroemulsion M2 | Lecithin-Mikroem. gem. Tabelle. 1 | 0,0 | 4,0 | - | - | - | - | - |
| | | | | | | | | |
| Rheocare XG® | Xanthan | - | - | - | 1,0 | 1,0 | 1,0 | 1,0 |
| Sodium Benzoate | | - | - | - | 0,5 | 0,5 | 0,5 | 0,5 |
| Dekafald® | DMDM Hydantoin | 0,2 | 0,2 | 0,2 | - | - | - | - |
| Citric Acid Sol. (50%) | | q.s. pH 5.5 | q.s. pH 5.5 | q.s. pH 5.5 | q.s. pH 4.7 | q.s. pH 4.7 | q.s. pH 4.7 | q.s. pH 4.7 |
| Aqua dem. | | add 100 | add 100 | add 100 | add 100 | add 100 | add 100 | add 100 |
| Restkämmbarkeit in % | | 93 | 77 | 78 | 65 | 56 | 89 | 75 |
| Aussehen nach 4 Wochen Lagerung bei RT | | Klar; stabil | Klar; stabil | Klar; stabil | Klar; stabil | Klar; stabil | Klar; stabil | Klar; stabil |

Tabelle 3 : Vergleichszubereitung mit den einzelnen Inhaltsstoffen der Mikroemulsionen M1 bzw. M2

| Ingredient | INCI | V5 [% AS] | V6 [% AS] | V7 [% AS] |
|---|---|---|---|---|
| Plantapon SF® | Sodium Cocoamphoacetate Glycerin Lauryl Glucoside Sodium Cocoyl Glutamate Sodium Lauryl Glucose Carboxylate | 16,8 | 16,8 | - |
| Sulfopon 1216G® | Sodium Coco-Sulfate | - | - | 9,0 |
| Plantacare 810 UF® | Capryl/Capryl Glucoside | - | - | 3,0 |

(fortgesetzt)

| | | V5 | V6 | V7 |
|---|---|---|---|---|
| Ingredient | INCI | [% AS] | [% AS] | [% AS] |
| Bestandteile der Mikroemulsion M1 einzeln; nicht vorformuliert | Lecithin-Mikroem. gem. Tabelle 1 | - | 4,0 | 4,0 |
| Bestandteile der Mikroemulsion M2 einzeln, nicht vorformuliert | Lecithin-Mikroem. gem. Tabelle. 1 | 4,0 | - | - |
| | | | | |
| Rheocare XG® | Xanthan | - | - | 1,0 |
| Sodium Benzoate | | - | - | 0,5 |
| Dekafald® | DMDM Hydantoin | 0,2 | 0,2 | - |
| Citric Acid Sol. (50%) | | q.s. pH 5.5 | q.s. pH 5.5 | q.s. pH 4.7 |
| Aqua dem. | | add 100 | add 100 | add 100 |
| Aussehen | | nicht transparent; rahmt nach 2 Stunden auf | nicht transparent; rahmt nach 2 Stunden auf | nicht transparent; rahmt nach 2 Stunden auf |

Die Konditionierleistung der Shampoos wurde anhand der Nasskämmbarkeit bestimmt; dazu wurden jeweils an 5 Haarsträhnen in einem automatisierten System folgende Behandlungen durchgeführt:
Die Vorbehandlung der Haarsträhnen (12cm/1g) der Firma IHIP wurde in einem automatisierten Haarbehandlungssystem durchgeführt:

- 15 min Reinigung mit 6% Natriumlaurylethersulfat (Aktivsubstanzgehalt), pH 6.5, danach intensives Spülen der Haare (3maliges Spülen für je 2 Minuten)
- 20 min Bleiche mit einer Lösung von 5 % Wasserstoffperoxid, pH 9.4 (mit Ammoniumhydroxidlösung eingestellt), danach intensives Spülen der Haare
- 30 min Trocknung in einem Luftstrom bei 55 °C

Direkt vor der Nullmessung wurden die Haare für 30 Minuten in Wasser bei 38 °C gequollen und anschließend mit einer automatischen Nassauskämmapparatur für 1 Minute ausgespült. Im automatisierten System zur Bestimmung der Nass- und Trockenkämmarbeit wurden die Kämmkräfte während 23 Kämmungen bestimmt und die Kämmarbeit durch Integration der gemessenen Kraft-Weg-Kurven errechnet. Nach der Nullmessung wurden die Haare sofort mit der Formulierung behandelt (0,25g/g Haar). Nach 5 Minuten Einwirkzeit wurde mit der automatischen Nassauskämmapparatur unter Standardbedingungen (38°C, 1l/Minute) gespült.
Die Behandlung und das folgende Ausspülen wurde ein zweites Mal wiederholt. Dann erfolgte die Vergleichsmessung (zur Nullmessung). Die Messungen wurden mit der feinen Kammseite der Naturkautschukkämme durchgeführt.
In der Tabelle findet sich der Mittelwert Restkämmbarkeit (%).
Die Restkämmarbeit pro Strähne wurde wie folgt berechnet:

Restkämmarbeit (%) = 100 x Kämmarbeit nach Produktbehandlung/Kämmarbeit vor Produktbehandlung.

Je niedriger der %-Wert desto besser ist die konditionierende Wirkung.
Die erfindungsgemäßen Haarshampoos H1, H3 und H4 zeigen bessere Konditionierleistungen als V1, V3 und V4 aufgrund der enthaltenen Mikroemulsion M1 bzw. M2 (siehe Tabelle 2).

Des Weiteren sind die erfindungsgemäßen Haarshampoos H2 und H4 klar und transparent und zeigen eine gute Lagerstabilität. Dagegen sind die Haarshampoos V5 bis V7 mit den gleichen Inhaltsstoffen, aber nicht in Form einer Mikroemulsion vorformuliert, instabil und zeigen ein Aufrahmen nach kurzer Zeit (vgl. H1 und H2 mit V5 und V6 bzw. H3 und H4 mit V7).

**Patentansprüche**

1.  Verwendung von einphasigen, transparenten Tensid/Öl/Wasser-Zubereitungen enthaltend

    (a) Alkyl- und/oder Alkenyloligoglykosid als Tensid
    (b) Lecithin als Co-Tensid
    (c) eine nicht-wasserlösliche organische Ölkomponente
    (d) und Wasser

    als konditionierend wirkendes Mittel in Reinigungsmittel für Haut und Haar.

2.  Verwendung nach Anspruch 1, **dadurch gekennzeichnet, dass** die Zubereitungen Alkyl- und/oder Alkenyloligoglykoside (a) gemäß der allgemeinen Formel $R^1O-[G]_p$ enthalten, in der $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3.  Verwendung nach mindestens einem der Ansprüche 1 bis 2, **dadurch gekennzeichnet, dass** sie als Lecithin (b) Rapslecithin, Sojalecithin, Maislecithin, Sonnenblumenlecithin und/oder deren Mischungen enthalten.

4.  Verwendung nach mindestens einem der Ansprüche 1 bis 3, **dadurch gekennzeichnet, dass** sie als Lecithin (b) Rapslecithin, vorzugsweise Rapslecithin mit einem Triglyceridanteil von 32 bis 42 Gew.-%- bezogen auf Rapslecithin- und insbesondere Rapslecithin mit einem Gehalt an - bezogen auf Gesamtgehalt Fettsäure im Triglycerid:

    Palmsäure: 4 bis 8 Gew.-%
    Stearinsäure: 0,5 bis 3 Gew.-%
    Ölsäure: 35 - 45 Gew.-%
    Linolsäure: 12 bis 22 Gew.-%
    Linolensäure: 2 bis 5 Gew.-%
    enthalten.

5.  Verwendung nach mindestens einem der Ansprüche 1 bis 4, **dadurch gekennzeichnet, dass** sie als organische Ölkomponente (c) verzweigte Fettalkohole, C6-$C_{22}$-Dialkylether, Esteröle, Kohlenwasserstoffe, lineare und ver-zweigte $C_6$-$C_{22}$-Dialkylcarbonate oder deren Mischungen enthalten.

6.  Verwendung nach mindestens einem der Ansprüche 1 bis 5, **dadurch gekennzeichnet, dass** sie als organische Ölkomponente (c) lineare C6-C12-Dialkylether und/oder lineare $C_6$-C12-Dialkylcarbonate, insbesondere Dicapryl-ether und/oder Dicaprylcarbonat, enthalten.

7.  Verwendung nach Anspruch 1, **dadurch gekennzeichnet, dass** sie ein weiteres Co-Tensid (b2) ausgewählt aus der Gruppe, die gebildet wird von Glycerinmonofettsäureester, Partialester von Polyglycerin mit durchschnittlichen Eigenkondensationsgraden von 2 bis 8, Partialester von Pentaerythrit und Partialester von Trimethylolpropanvorzugsweise Glycerinmonooleat, enthalten können.

8.  Verwendung nach mindestens einem der Ansprüche 1 bis 7, **dadurch gekennzeichnet, dass** die Zubereitungen in Mengen von

    5 bis 25 Gew.-%, vorzugsweise 5 bis 20 Gew.-% der Komponente (a)
    5 bis 25 Gew.-%, vorzugsweise 7,5 bis 20 Gew.-% der Komponente (b)
    0 bis 15 Gew.-% der Komponente (b2)
    5 bis 50 Gew.-%, vorzugsweise 15 bis 30 Gew.-% der Komponente (c) und
    0 bis 10 Gew.-% weitere Inhaltsstoffe sowie
    ad 100 Gew.-% Wasser als Komponente (d) enthalten - bezogen auf die verwendete Zubereitung.

**9.** Verwendung nach Anspruch 8, **dadurch gekennzeichnet, dass** die verwendeten Zubereitungen in Mengen von

5 bis 15 Gew.-% Komponente (a)
10 bis 25 Gew.-% Komponente (b)
15 bis 25 Gew.-% Komponente (c) und
0 bis 10 Gew.-% weitere Inhaltsstoffe sowie
ad 100 Gew.-% Wasser als Komponente (d) enthalten - bezogen auf die verwendete Zubereitung.

**10.** Verwendung nach Anspruch 8, **dadurch gekennzeichnet, dass** die verwendeten Zubereitungen in Mengen von

10 bis 20 Gew.-% der Komponente (a)
5 bis 15 Gew.-% der Komponente (b)
5 bis 15 Gew.-% der Komponente (b2)
15 bis 25 Gew.-% der Komponente (c) und
0 bis 10 Gew.-% weitere Inhaltsstoffe sowie
ad 100 Gew.-% Wasser als Komponente (d) enthalten - bezogen auf die verwendete Zubereitung.

**11.** Verwendung nach mindestens einem der Ansprüche 8 bis 10, **dadurch gekennzeichnet, dass** in den verwendeten Zubereitungen weitere Inhaltsstoffe enthalten sind, die ausgewählt sind aus der Gruppe, die gebildet wird von Hydrotope, Konservierungsmittel, Antioxidantien, biogene Wirkstoffe, Biozide und pH-Regulantien.

**12.** Verwendung der Zubereitungen nach mindestens einem der Ansprüche 1 bis 11 als konditionierend wirkendes Mittel, insbesondere zur Verbesserung der Kämmbarkeit, der Glätte, der Geschmeidigkeit und des Glanzes der menschlichen Haare.

**13.** Verwendung der transparenten Zubereitungen nach mindestens einem der Ansprüche 1 bis 11, **dadurch gekennzeichnet, dass** die konditionierend wirkenden Mittel in Form einer Vorformulierung in den kosmetischen Reinigungsmitteln, vorzugsweise in Haarshampoos, verwendet werden.

**14.** Einphasige, transparente Tensid/Öl/Wasser-Zubereitungen für Reinigungsmittel für Haut und Haar, **dadurch gekennzeichnet, dass** sie in Mengen von

a) 5 bis 25 Gew.-%, vorzugsweise 5 bis 20 Gew.-% Alkyl- und/oder Alkenyloligoglykosid als Tensid
b) 5 bis 25 Gew.-%, vorzugsweise 7,5 bis 20 Gew.-% Lecithin als Co-Tensid
b2) 0 bis 15 Gew.-% ein weiteres Co-Tensid ausgewählt aus der Gruppe, die gebildet wird von Glycerinmonofettsäureester, Partialester von Polyglycerin mit durchschnittlichen Eigenkondensationsgraden von 2 bis 8, Partialester von Pentaerythrit und Partialester von Trimethylolpropan
c) 5 bis 50 Gew.-%, vorzugsweise 15 bis 30 Gew.-% eine nicht-wasserlösliche organischen Ölkomponente und
0 bis 10 Gew.-% weiterer Inhaltsstoffe sowie
d) ad 100 Gew.-% Wasser als Komponente d) - Gew.-% bezogen auf Zubereitung - enthalten.

**15.** Milde kosmetische Reinigungsmittel für Haut und Haar enthaltend

A) konditionierend wirkende einphasige, transparente Tensid/Öl/Wasser-Zubereitungen in Mengen von
a) 5 bis 25 Gew.-%, vorzugsweise 5 bis 20 Gew.-% Alkyl- und/oder Alkenyloligoglykosid als Tensid
b) 5 bis 25 Gew.-%, vorzugsweise 7,5 bis 20 Gew.-% Lecithin als Co-Tensid
b2) 0 bis 15 Gew.-% ein weiteres Co-Tensid ausgewählt aus der Gruppe, die gebildet wird von Glycerinmonofettsäureester, Partialester von Polyglycerin mit durchschnittlichen Eigenkondensationsgraden von 2 bis 8, Partialester von Pentaerythrit und Partialester von Trimethylolpropan
c) 5 bis 50 Gew.-%, vorzugsweise 15 bis 30 Gew.-% eine nicht-wasserlösliche organischen Ölkomponente und
0 bis 10 Gew.-% weiterer Inhaltsstoffe sowie
d) ad 100 Gew.-% Wasser als Komponente d) - Gew.-% bezogen auf Zubereitung
B) anionische Tenside sowie
C) ggf. nichtionische, zwitterionische und/oder amphotere Tenside und
D) ggf. weitere kosmetische Zusatzstoffe und/oder Wasser.

**16.** Verfahren zur Herstellung von milden kosmetischen Reinigungsmitteln für Haut und Haar, nach Anspruch 15 **dadurch gekennzeichnet, dass** die transparente Tensid/Öl/ Wasser-Zubereitung A) in die vorgelegten weiteren In-

**EP 3 250 178 B1**

haltsstoffe B) sowie ggf. C) und D) und ggf. Wasser bei Raumtemperatur eingerührt wird.

**Claims**

1. The use of single-phase, transparent surfactant/ oil/water preparations comprising

   (a) alkyl and/or alkenyl oligoglycoside as surfactant
   (b) lecithin as co-surfactant
   (c) a water-insoluble organic oil component
   (d) and water

   as a conditioning composition in cleansing compositions for skin and hair.

2. The use according to claim 1, **wherein** the preparations comprise alkyl and/or alkenyl oligoglycosides (a) according to the general formula $R^1O-[G]_p$, in which $R^1$ is an alkyl and/or alkenyl radical having 4 to 22 carbon atoms, G is a sugar radical having 5 or 6 carbon atoms and p is a number from 1 to 10.

3. The use according to at least one of claims 1 to 2, **wherein** as lecithin (b), said compositions comprise rapeseed lecithin, soya lecithin, maize lecithin, sunflower lecithin and/or mixtures thereof.

4. The use according to at least one of claims 1 to 3, **wherein** as lecithin (b), said compositions comprise rapeseed lecithin, preferably rapeseed lecithin having a triglyceride fraction of 32 to 42% by weight, based on rapeseed lecithin, and especially rapeseed lecithin having a content of - based on the total fatty acid content in the triglyceride:

   palmitic acid: 4 to 8% by weight
   stearic acid: 0.5 to 3% by weight
   oleic acid: 35-45% by weight
   linoleic acid: 12 to 22% by weight
   linolenic acid: 2 to 5% by weight.

5. The use according to at least one of claims 1 to 4, **wherein** as organic oil component (c), said compositions comprise branched fatty alcohols, $C_6$-$C_{22}$-dialkyl ethers, ester oils, hydrocarbons, linear and branched $C_6$-$C_{22}$-dialkyl carbonates or mixtures thereof.

6. The use according to at least one of claims 1 to 5, **wherein** as organic oil component (c), said compositions comprise linear $C_6$-$C_{12}$-dialkyl ethers and/or linear $C_6$-$C_{12}$-dialkyl carbonates, especially dicaprylyl ether and/or dicaprylyl carbonate.

7. The use according to claim 1, **wherein** said compositions may comprise a further co-surfactant (b2) selected from the group formed from glycerol monofatty acid esters, partial esters of polyglycerol having average degrees of selfcondensation of 2 to 8, partial esters of pentaerythritol and partial esters of trimethylolpropane, preferably glycerol monooleate.

8. The use according to at least one of claims 1 to 7, **wherein** the preparations comprise amounts of

   5 to 25% by weight, preferably 5 to 20% by weight of component (a)
   5 to 25% by weight, preferably 7.5 to 20% by weight of component (b)
   0 to 15% by weight of component (b2)
   5 to 50% by weight, preferably 15 to 30% by weight of component (c) and
   0 to 10% by weight further ingredients and
   to 100% by weight water as component (d) - based on the preparation used.

9. The use according to claim 8, **wherein** the preparations used comprise amounts of

   5 to 15% by weight component (a)
   10 to 25% by weight component (b)
   15 to 25% by weight component (c) and

16

0 to 10% by weight further ingredients and also
to 100% by weight water as component (d) - based on the preparation used.

10. The use according to claim 8, **wherein** the preparations used comprise amounts of

10 to 20% by weight of component (a)
5 to 15% by weight of component (b)
5 to 15% by weight of component (b2)
15 to 25% by weight of component (c) and
0 to 10% by weight further ingredients and also
to 100% by weight water as component (d) - based on the preparation used.

11. The use according to at least one of claims 8 to 10, **wherein** further ingredients are present in the preparations used which are selected from the group formed by hydrotropes, preservatives, antioxidants, biogenic active ingredients, biocides and pH regulators.

12. The use of the preparations according to at least one of claims 1 to 11 as conditioning compositions, especially to improve combability, smoothness, softness and gloss of human hair.

13. The use of the transparent preparations according to at least one of claims 1 to 11, **wherein** the conditioning compositions are used in the form of a pre-formulation in cosmetic cleansing compositions, preferably in hair shampoos.

14. A single-phase, transparent surfactant/oil/water preparation for cleansing compositions for skin and hair, **wherein** said preparations comprise amounts of

a) 5 to 25% by weight, preferably 5 to 20% by weight alkyl and/or alkenyl oligoglycoside as surfactant
b) 5 to 25% by weight, preferably 7.5 to 20% by weight lecithin as co-surfactant
b2) 0 to 15% by weight of a further co-surfactant selected from the group formed from glycerol monofatty acid esters, partial esters of polyglycerol having average degrees of self-condensation of 2 to 8, partial esters of pentaerythritol and partial esters of trimethylolpropane
c) 5 to 50% by weight, preferably 15 to 30% by weight of a water-insoluble organic oil component and 0 to 10% further ingredients and
d) to 100% by weight water as component d) - % by weight based on the preparation.

15. A mild cosmetic cleansing composition for skin and hair comprising

A) conditioning single-phase, transparent surfactant/oil/water preparations in amounts of
a) 5 to 25% by weight, preferably 5 to 20% by weight alkyl and/or alkenyl oligoglycoside as surfactant
b) 5 to 25% by weight, preferably 7.5 to 20% by weight lecithin as co-surfactant
b2) 0 to 15% by weight of a further co-surfactant selected from the group formed from glycerol monofatty acid esters, partial esters of polyglycerol having average degrees of self-condensation of 2 to 8, partial esters of pentaerythritol and partial esters of trimethylolpropane
c) 5 to 50% by weight, preferably 15 to 30% by weight of a water-insoluble organic oil component and 0 to 10% by weight further ingredients and
d) to 100% by weight water as component d) - % by weight based on the preparation
B) anionic surfactants and
C) optionally non-ionic, zwitterionic and/or amphoteric surfactants and
D) optionally further cosmetic additives and/or water.

16. A method for producing mild cosmetic cleansing compositions for skin and hair according to claim 15, **wherein** the transparent surfactant/oil/water preparation A) is stirred at room temperature into the initially introduced further ingredients B) and also optionally C) and D) and optionally water.

**Revendications**

1. Utilisation de préparations tensioactif/huile/eau transparentes monophasées contenant :

(a) un alkyl- et/ou alcényloligoglycoside en tant que tensioactif,
(b) une lécithine en tant que co-tensioactif,
(c) un composant huileux organique non soluble dans l'eau,
(d) et de l'eau,

en tant qu'agent à effet conditionnant dans des détergents pour la peau et les cheveux.

**2.** Utilisation selon la revendication 1, **caractérisée en ce que** les préparations contiennent des alkyl- et/ou alcényloligoglycosides (a) selon la formule générale $R^1O$-$[G]_p$, dans laquelle $R^1$ représente un radical alkyle et/ou alcényle de 4 à 22 atomes de carbone, G représente un radical de sucre contenant 5 ou 6 atomes de carbone, et p représente des nombres de 1 à 10.

**3.** Utilisation selon au moins l'une quelconque des revendications 1 à 2, **caractérisée en ce que** de la lécithine de colza, de la lécithine de soja, de la lécithine de maïs, de la lécithine de tournesol et/ou leurs mélanges sont contenus en tant que lécithine (b).

**4.** Utilisation selon au moins l'une quelconque des revendications 1 à 3, **caractérisée en ce que** de la lécithine de colza, de préférence de la lécithine de colza ayant une proportion de triglycéride de 32 à 42 % en poids, par rapport à la lécithine de colza, et notamment de la lécithine de colza ayant une teneur en, par rapport à la teneur totale en acides gras dans le triglycéride :

acide palmitique : 4 à 8 % en poids,
acide stéarique : 0,5 à 3 % en poids,
acide oléique : 35 à 45 % en poids,
acide linoléique : 12 à 22 % en poids,
acide linolénique : 2 à 5 % en poids,
est contenue en tant que lécithine (b).

**5.** Utilisation selon au moins l'une quelconque des revendications 1 à 4, **caractérisée en ce que** des alcools gras ramifiés, des éthers dialkyliques en $C_6$-$C_{22}$, des huiles d'esters, des hydrocarbures, des carbonates de dialkyle en $C_6$-$C_{22}$ linéaires et ramifiés ou leurs mélanges sont contenus en tant que composant huileux organique (c).

**6.** Utilisation selon au moins l'une quelconque des revendications 1 à 5, **caractérisée en ce que** des éthers dialkyliques en $C_6$-$C_{12}$ linéaires et/ou des carbonates de dialkyle en $C_6$-$C_2$ linéaires, notamment l'éther dicaprylique et/ou le carbonate dicaprylique, sont contenus en tant que composant huileux organique (c).

**7.** Utilisation selon la revendication 1, **caractérisée en ce qu'**un co-tensioactif supplémentaire (b2) choisi dans le groupe qui est formé par les esters de monoacides gras de glycérine, les esters partiels de polyglycérine présentant des degrés de condensation intrinsèque moyens de 2 à 8, les esters partiels de pentaérythrite et les esters partiels de triméthylolpropane, de préférence le monooléate de glycérine, peut être contenu.

**8.** Utilisation selon au moins l'une quelconque des revendications 1 à 7, **caractérisée en ce que** les préparations contiennent en quantités de :

5 à 25 % en poids, de préférence 5 à 20 % en poids, le composant (a),
5 à 25 % en poids, de préférence 7,5 à 20 % en poids, le composant (b),
0 à 15 % en poids le composant (b2),
5 à 50 % en poids, de préférence 15 à 30 % en poids, le composant (c), et
0 à 10 % en poids d'autres constituants, ainsi que jusqu'à 100 % en poids d'eau en tant que composant (d),
par rapport à la préparation utilisée.

**9.** Utilisation selon la revendication 8, **caractérisée en ce que** les préparations utilisées contiennent en quantités de :

5 à 15 % en poids le composant (a),
10 à 25 % en poids le composant (b),
15 à 25 % en poids le composant (c), et
0 à 10 % en poids d'autres constituants, ainsi que jusqu'à 100 % en poids d'eau en tant que composant (d),
par rapport à la préparation utilisée.

EP 3 250 178 B1

**10.** Utilisation selon la revendication 8, **caractérisée en ce que** les préparations utilisées contiennent en quantités de :

10 à 20 % en poids le composant (a),
5 à 15 % en poids le composant (b),
5 à 15 % en poids le composant (b2),
15 à 25 % en poids le composant (c), et
0 à 10 % en poids d'autres constituants, ainsi que jusqu'à 100 % en poids d'eau en tant que composant (d), par rapport à la préparation utilisée.

**11.** Utilisation selon au moins l'une quelconque des revendications 8 à 10, **caractérisée en ce que** d'autres constituants sont contenus dans les préparations utilisées, qui sont choisis dans le groupe qui est formé par les hydrotopes, les conservateurs, les antioxydants, les agents actifs biogènes, les biocides et les régulateurs de pH.

**12.** Utilisation des préparations selon au moins l'une quelconque des revendications 1 à 11 en tant qu'agent à effet conditionnant, notamment pour améliorer l'aptitude au démêlage, le lissage, la souplesse et la brillance de cheveux humains.

**13.** Utilisation des préparations transparentes selon au moins l'une quelconque des revendications 1 à 11, **caractérisée en ce que** les agents à effet conditionnant sont utilisés sous la forme d'une pré-formulation dans les détergents cosmétiques, de préférence dans des shampoings capillaires.

**14.** Préparations tensioactif/huile/eau transparentes monophasées pour détergents pour la peau et les cheveux, **caractérisées en ce qu'**elles contiennent en quantités de :

a) 5 à 25 % en poids, de préférence 5 à 20 % en poids, un alkyl- et/ou alcényloligoglycoside en tant que tensioactif,
b) 5 à 25 % en poids, de préférence 7,5 à 20 % en poids, une lécithine en tant que co-tensioactif,
b2) 0 à 15 % en poids un co-tensioactif supplémentaire choisi dans le groupe qui est formé par les esters de monoacides gras de glycérine, les esters partiels de polyglycérine présentant des degrés de condensation intrinsèque moyens de 2 à 8, les esters partiels de pentaérythrite et les esters partiels de triméthylolpropane,
c) 5 à 50 % en poids, de préférence 15 à 30 % en poids, un composant huileux organique non soluble dans l'eau, et
0 à 10 % en poids d'autres constituants, ainsi que
d) jusqu'à 100 % en poids d'eau en tant que composant d), les % en poids se rapportant à la préparation.

**15.** Détergents cosmétiques doux pour la peau et les cheveux, contenant :

A) des préparations tensioactif/huile/eau transparentes monophasées à effet conditionnant, qui contiennent en quantités de
a) 5 à 25 % en poids, de préférence 5 à 20 % en poids, un alkyl- et/ou alcényloligoglycoside en tant que tensioactif,
b) 5 à 25 % en poids, de préférence 7,5 à 20 % en poids, une lécithine en tant que co-tensioactif,
b2) 0 à 15 % en poids un co-tensioactif supplémentaire choisi dans le groupe qui est formé par les esters de monoacides gras de glycérine, les esters partiels de polyglycérine présentant des degrés de condensation intrinsèque moyens de 2 à 8, les esters partiels de pentaérythrite et les esters partiels de triméthylolpropane,
c) 5 à 50 % en poids, de préférence 15 à 30 % en poids, un composant huileux organique non soluble dans l'eau, et
0 à 10 % en poids d'autres constituants, ainsi que
d) jusqu'à 100 % en poids d'eau en tant que composant d), les % en poids se rapportant à la préparation,
B) des tensioactifs anioniques, ainsi que
C) éventuellement des tensioactifs non ioniques, zwitterioniques et/ou amphotères, et
D) éventuellement des additifs cosmétiques supplémentaires et/ou de l'eau.

**16.** Procédé de fabrication de détergents cosmétiques doux pour la peau et les cheveux selon la revendication 15, **caractérisé en ce que** la préparation tensioactif/huile/eau transparente A) est incorporée dans les autres constituants chargés initialement B), ainsi qu'éventuellement C) et D) et éventuellement de l'eau à température ambiante.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008155075 A
- WO 2011116881 A
- WO 2013149853 A
- US 2013012423 A
- WO 08155075 A1

**In der Beschreibung aufgeführte Nicht-Patentliteratur**

- **E. ACOSTA et al.** *Environ. Sci. Technol.,* 2005, vol. 39, 1275-1282
- **F. PATTARINO et al.** *J.Dispersion Science and Technology,* 2000, vol. 21 (3), 345-263
- **J. SCHWARZ et al.** *European Journal of Pharmaceutics and Biopharmaceutics,* 2012, vol. 81, 557-562
- *Official Journal of the European Union,* 20. September 2008
- **CLAUDIA HEIFT.** Institut für Biochemie und Lebensmittelchemie. Universität Hamburg, 2007